# EUROPEAN PATENT SPECIFICATION

(11) **EP 3 081 252 B1**
(45) Date of publication and mention of the grant of the patent: **22.08.2018**
(21) Application number: 14869555.4
(22) Date of filing: 30.10.2014
(51) Int. Cl.: A61M 29/02, A61M 25/10, A61B 17/56, A61B 17/88, A61B 17/16

(54) **VERTEBRAL BALLOON DILATION SYSTEM**
ERWEITERUNGSSYSTEM FÜR VERTEBRALEN BALLON
SYSTÈME DE DILATATION VERTÉBRALE À BALLONNET

(30) Priority: 12.12.2013 CN 201310671713
(43) Date of publication of application: 19.10.2016
(73) Proprietor: Ningbo Hicren Biotechnology Co. Ltd., Ningbo, Zhejiang 315336 (CN)
(72) Inventor: LV, Shiwen, Ningbo Zhejiang 315336 (CN); WANG, Yu, Ningbo Zhejiang 315336 (CN); MAO, Keya, Ningbo Zhejiang 315336 (CN); XIN, Chaohua, Ningbo Zhejiang 315336 (CN)
(74) Representative: Haseltine Lake LLP
(86) International application number: PCT/CN2014/089873
(87) International publication number: WO 2015/085837

(56) References cited:
- WO-A1-2009/065085
- WO-A1-2012/134592
- CN-A- 101 909 532
- CN-A- 102 499 740
- CN-A- 102 784 434
- CN-A- 103 690 228
- CN-Y- 2 905 092
- GB-A- 2 046 096
- US-A- 5 972 015
- US-A1- 2009 299 282
- US-A1- 2012 016 369
- US-A1- 2012 165 732

## Description

### Technical Field

The present disclosure relates to the field of medical equipment, and more particularly, to a vertebral balloon dilation system for use in percutaneous kyphoplasty (PKP).

### Background

Osteoporotic vertebral compression fracture is a common disease harmful to the health of middle-aged and elderly people. For vertebral compression fractures, traditional treatment methods are conservative for the most part and include bed rest, drug analgesia, and external fixation with orthosis, etc.. These traditional treatment methods often result in a further loss of bone mass and an exacerbation of osteoporosis, thus forming a vicious cycle, while an open surgery can easily result in failures of internal fixation due to the poor health condition of the patient or insufficient fixation strength of screws. WO 2012/134592 describes an apparatus assembly comprising a conforming catheter shaft an expandable element having an expandable balloon portion bonded at the distal end of the catheter shaft, and a fluid passageway extending from a proximal end of the catheter shaft to the interior of the balloon portion of the expandable element. In 1987, French doctors, Deramond et al., reported that good curative effects had been achieved for treating aggressive hemangioma of C2 vertebral body by percutaneous vertebroplasty (PVP). Hereafter, such technology is also applied in treatment of the malignant vertebral tumor and osteoporosis. The drawbacks of such surgery include that it is only possible to fix the injured vertebral body with deformity and alleviate the pain, but impossible to restore the vertebral height or correct the kyphotic deformity. Moreover, since the low-viscosity bone cement is injected into the vertebral cancellous bone directly under a relatively higher pressure, it is difficult to control the flow, and the leakage rate of the bone cement is higher, which is within the range of 30% - 67%, as reported in references. By 1994, American scholars, Reiley et al., designed a new technique for correcting kyphotic deformity by means of balloon dilation based on PVP, and such technique was developed as the percutaneous kyphoplasty (PKP) and was approved by the FDA in 1998 for clinical applications. The technique includes the following steps: inserting a dilatable balloon into a collapsed vertebral body by percutaneous puncture, lifting up the endplate by the dilation of the balloon, restoring the height of the vertebral body and correcting the kyphotic deformity, thereby forming a hollow cavity surrounded by the bone shell in the vertebral body and injecting it with the high-viscosity bone cement under a relatively lower pressure. In order to insert the balloon into the vertebral body smoothly, a working channel has to be established primarily with a puncture needle and a working sleeve tube, then a working cavity for the un-dilated balloon is drilled with a bone drill, and finally the balloon is inserted into the vertebral body. The surgical procedure is complex. Because of the structure of the vertebral body, the balloon dilation has to be operated at both sides of each vertebral body in order to maintain the biomechanical force balance of the vertebral body. Furthermore, the surgery has to be operated under X-ray monitoring, therefore, the more complicated the surgical procedures, the longer the time spent, the greater the damage to the health of the doctor, and the higher the level of physical strength of the doctor required. The balloon dilation operated at both sides of the vertebral body may thus result in a greater trauma to the patient a higher incidence of complications, and may increase the economical burden of the patient. The above defects will become more significant in the case of multi-segmental vertebral lesions.

### Summary

The present disclosure aims to provide a vertebral balloon dilation system, which can be fed to the center or to the opposite side of the vertebral body simply by puncturing at only one side without drilling with a bone drill to form a cavity before feeding the dilatable balloon. According to the present disclosure, a dilatable balloon is fed to the center or the opposite side of the vertebral body with a liner core provided with a curved section at its distal end, and then a developer solution is injected into the balloon by means of a pushing tube, thereby simplifying surgical procedures, shortening the duration of surgery, reducing the incidence of complications, alleviating the pain of patients, and reducing the economical burden of patients.

An objective of the present invention is realized by the following technical scheme:
A vertebral balloon dilation system comprises a pushing tube and a balloon fixedly connected to a distal end of said pushing tube, wherein said vertebral balloon dilation system can withstand water pressure of no less than 10 atm; said pushing tube is a rigid tube, a lumen of said pushing tube is in communication with an interior of said balloon; and said balloon is made of a membrane material, a distal end of said balloon is fixedly connected with a sealing head that is closed at its distal end and has a blind hole at its proximal end, wherein said blind hole has an opening towards the interior of said balloon. Said vertebral balloon dilation system further comprises a sleeve tube and a liner core, wherein said liner core is slidably inserted into said pushing tube, a distal end part of said liner core is a curved section, which protrudes out of the distal end of said pushing tube, the distal end of said liner core is inserted into said blind hole of said sealing head through said pushing tube and the interior of said balloon, and said balloon is configured to wrap up the curved section of the liner core; a distal end part of said sleeve tube is a flexible section, whereas a proximal end part of said sleeve tube is a rigid section, wherein said flexible section is rigid in the radial direction and said sleeve tube is sleeved on said pushing tube and said balloon; and, when said vertebral balloon dilation system is operated, said sleeve tube slides towards a proximal end of said pushing tube in the axial direction, and said balloon is exposed.

Objectives of the present invention can be further realized by the following technical schemes:
In some embodiments, said sealing head is connected with said balloon in a connection manner selected from binding connection, threaded connection, compression connection, adhesive connection, or welding connection or combinations thereof.

In some embodiments, said membrane material is PTFE membrane.

In some embodiments, said sealing head is made of a rigid material, the distal end of which is in a sharp shape, whereas the distal end of said sleeve tube is sleeved on or connected with said sealing head.

In some embodiments, an end part of said curved section of said liner core is a straight section, which is inserted into said blind hole in said sealing head and reaches the bottom of said blind hole.

In some embodiments, said flexible section of said sleeve tube is cut from a metal tube, formed of a spring, made of a hard medical polymer material, or formed of any combinations of metal materials and polymer materials.

In some embodiments, said balloon is in a banana shape or a dumbbell shape.

In some embodiments, said balloon is foldable in its axial direction, and is configured to wrap up said curved section of the liner core along its circumferential direction.

In some embodiments, said vertebral balloon dilation system further comprises a liner core handle fixedly connected to the proximal end of said liner core, a pushing handle fixedly connected to the proximal end of said pushing tube, and a sleeve tube handle connected to the proximal end of said sleeve tube, wherein said pushing handle includes one or more guide grooves, a distal end of said pushing handle is provided with a stop block for limiting the position of said sleeve tube handle, and said sleeve tube handle is provided with one or more guide blocks engaged with said guide grooves such that while said sleeve tube handle is moved, said sleeve tube will move in the axial direction together with said sleeve tube handle.

In some embodiments, the distal end of said pushing tube and said balloon are fixedly connected by means of hot melt welding, or sintering the proximal end of said balloon entirely to an outside of said pushing tube.

As compared with the prior art, the present disclosure has the following characteristics and advantages:
1. In a surgical procedure with a prior art balloon, each vertebral body needs to be punctured and dilated at both sides. The vertebral balloon dilation system of the present disclosure is provided with a curved section at its distal end so as to feed the balloon to the center or the opposite side of the vertebral body, such that the dilation at both sides can be achieved by puncturing at only one side, thereby effectively reducing the trauma to the patient, shortening the duration of surgery, reducing the incidence of complications and meanwhile reducing the economical burden of the patient, and such advantages are more apparent in the surgery for multi-segmental vertebral lesions.
2. In the prior art, before using a balloon, a channel for feeding the balloon has to be drilled with a bone drill. In view of the defect of complicated procedures, the balloon of the present disclosure is provided with a hard sealing head at the distal end thereof, and furthermore, a rigid sleeve tube is sleeved on the balloon, such that when feeding the balloon to the vertebral body, it is unnecessary to drill a working cavity for the balloon in advance like that in the traditional PKP surgery. Due to the protection effect of the sleeve tube on the balloon, the balloon can be fed into the vertebral body directly such that the surgical procedures are simplified and the risk of surgery is lowered, and due to the reinforcing effect of the liner core, the procedure of inserting the vertebral balloon dilation system into the vertebral body can be implemented more reliably and conveniently.
3. The flexible section of the sleeve tube of the present disclosure is rigid in the radial direction, such that it can withstand a certain pressure and the bone tissue can be pushed away by the curved section by rotating the handle so as to feed the balloon to the vertebral body more smoothly.
4. According to the anatomical knowledge, the vertebral body is approximately in an oval shape, and vertebral compression fractures basically occur at the front edge of the vertebral body; in some embodiments, the balloon of the present disclosure is in a banana shape or a dumbbell shape so as to be better conform to the shape of the leading edge of the vertebral body, and better maintain the original biomechanical balance of the vertebral body after the bone cement is solidified.

### Brief Description of Drawings

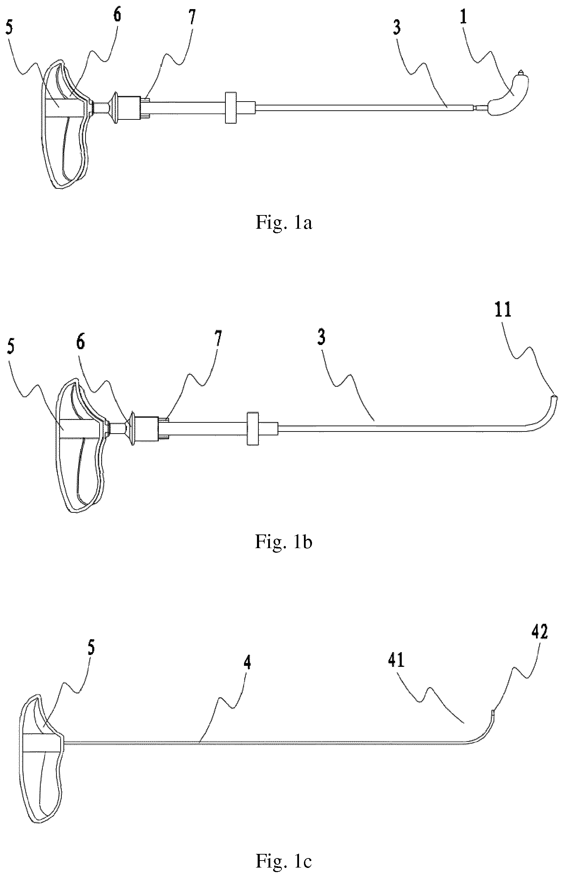

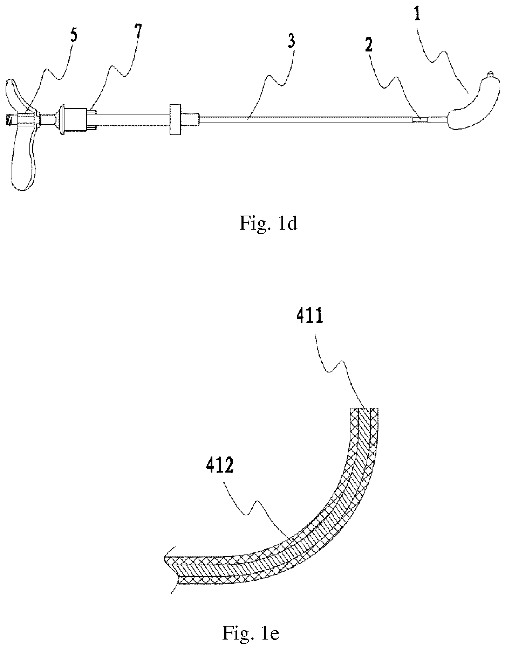

Fig. 1 shows structural schematic diagrams illustrating one embodiment of the present invention, wherein Fig. 1a is a structural schematic diagram when the balloon is in a full filling state, Fig. 1b is a structural schematic diagram when the vertebral balloon dilation system is in the initial state, Fig. 1c is a structural schematic diagram illustrating the liner core and the liner core handle, Fig. Id is a schematic diagram illustrating the vertebral balloon dilation system in the state when the liner core has been withdrawn, and Fig. 1e is a structural schematic diagram illustrating the curved section of the liner core, wherein the curved section is made of nitinol wires and polymer material.

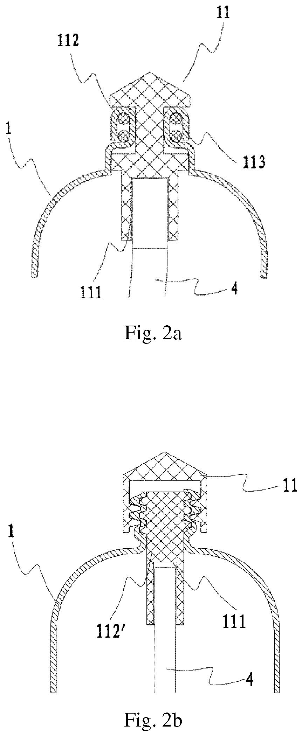

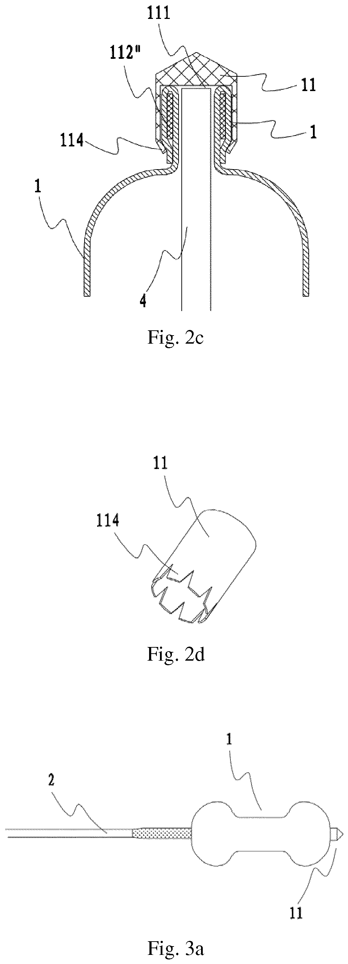

Fig. 2 shows structural schematic diagrams illustrating the sealing head in the present disclosure, wherein Fig. 2a is a structural schematic diagram illustrating the sealing head connected with the balloon by means of a binding connection, Fig. 2b is a structural schematic diagram illustrating the sealing head connected with the balloon by means of a threaded connection, Fig. 2c is a structural schematic diagram illustrating the sealing head connected with the balloon by means of a compression connection, and Fig. 2d is a structural schematic diagram illustrating a compression piece for the compression connection of the sealing head and the balloon as shown in Fig. 2c.

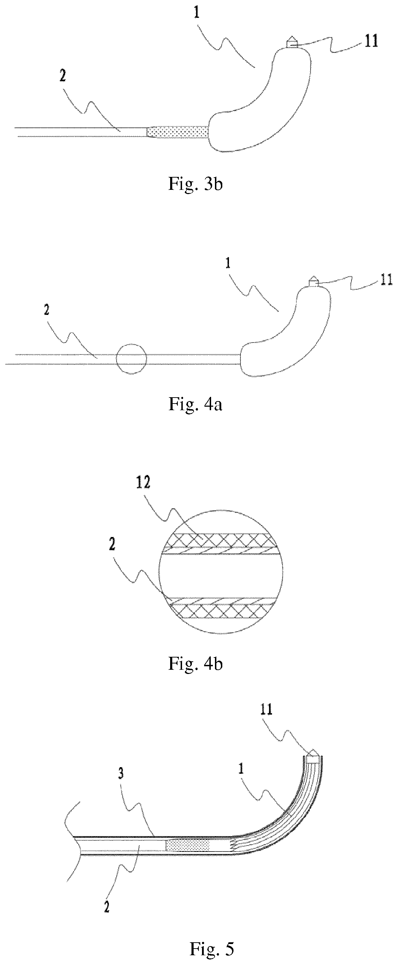

Fig. 3 shows schematic diagrams of the balloon according to one embodiment of the present invention, wherein Fig. 3a is a schematic diagram of the balloon in a dumbbell shape, and Fig. 3b is a schematic diagram of the balloon in a banana shape.
Fig. 4 shows structural schematic diagrams illustrating the fixed connection between the proximal end of the balloon and the distal end of the pushing tube according to one embodiment of the present invention, wherein Fig. 4a is a schematic diagram illustrating the proximal end part of the balloon sintered to the external surface of the pushing tube, and Fig. 4b is a cutaway view of Fig. 4a.
Fig. 5 is a schematic diagram illustrating the relative positions of the sleeve tube, the pushing tube, the liner core and the balloon when the vertebral balloon dilation system is in the initial state according to one embodiment of the present invention.

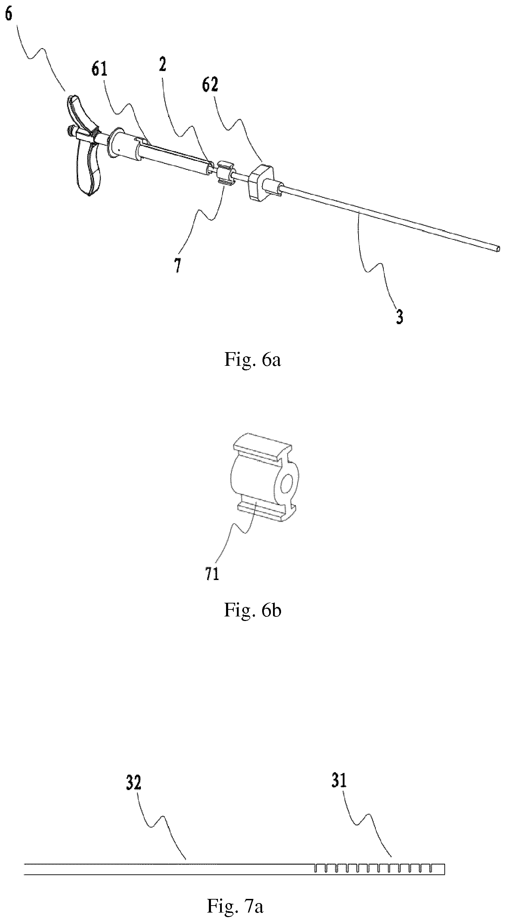

Fig. 6 shows structural schematic diagrams of the pushing handle and the sleeve tube handle according to one embodiment of the present invention, wherein Fig. 6a is a schematic diagram illustrating the assembly relation of the pushing handle and the sleeve tube handle, and Fig. 6b is a structural schematic diagram of a sliding block.

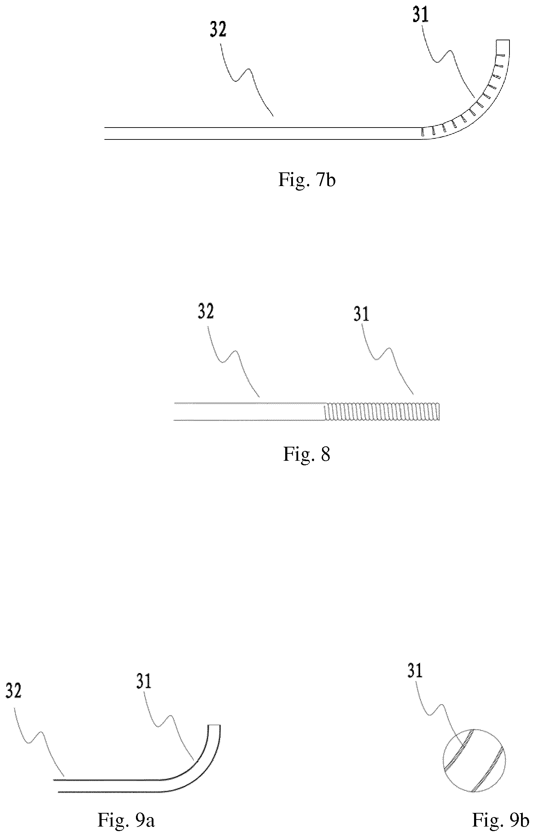

Fig. 7 shows structural schematic diagrams of the sleeve tube according to one embodiment of the present invention, wherein Fig. 7a is a schematic diagram illustrating the straight state of the sleeve tube cut from a metal tube, and Fig. 7b is a schematic diagram illustrating the curved state of the sleeve tube cut from a metal tube.
Fig. 8 is a structural schematic diagram of the sleeve tube according to another embodiment of the present invention.
Fig. 9 shows local cutaway views of the sleeve tube according to a third embodiment of the present invention, wherein Fig. 9a is a structural schematic diagram of the sleeve tube made of a medical polymer material, and Fig. 9b is a partial enlarged view of the local cutaway view as shown in Fig. 9a.

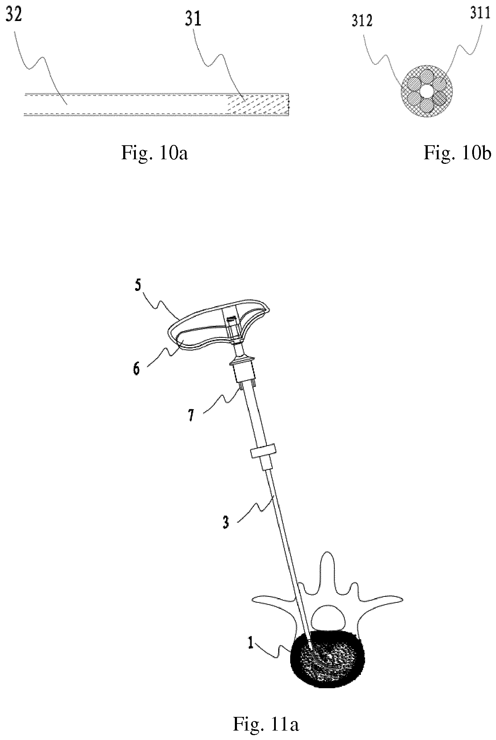

Fig. 10 shows structural schematic diagrams of the sleeve tube according to a fourth embodiment of the present invention, wherein Fig. 10a is a structural schematic diagram of the sleeve tube made of a metal material and a medical polymer material, and Fig. 10b is a cross-section view of the flexible section of the sleeve tube as shown in Fig. 10a.

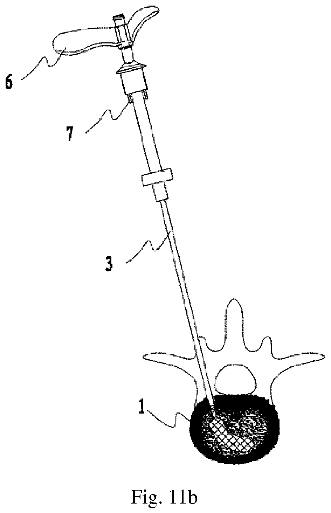

Fig. 11 shows schematic diagrams illustrating the in-use state of the vertebral balloon dilation system according to one embodiment of the present invention, wherein Fig. 11a is a schematic diagram illustrating the in-use state when the balloon is exposed after being fed to the vertebral body, and Fig. 11b is a schematic diagram illustrating the in-use state after injecting the bone cement to the balloon.

### Detailed Description of Disclosed Embodiments

In order to make the objectives, technical schemes and advantages of the present disclosure more apparent and better understood, the present disclosure will be described in more detail with reference to the accompanying figures and embodiments.

The proximal end, as described in the present disclosure, refers to the end near to the surgical operator, and the distal end refers to the end far away from the surgical operator.

As shown in Figs. 1a, 1b, 1c and Id, according to one embodiment of the present invention, a vertebral balloon dilation system comprises a balloon 1, a pushing tube 2 fixedly connected to the proximal end of the balloon 1, a sleeve tube 3, and a liner core 4; the vertebral balloon dilation system can withstand the water pressure of no less than 10 atm; the pushing tube 2 is a rigid tube, the lumen of the pushing tube 2 is in communication with the interior of the balloon 1; the balloon 1 is made of a membrane material, the distal end of the balloon 1 is fixedly connected with a sealing head 11, the sealing head 11 is closed at its distal end and has a blind hole 111 at its proximal end, the blind hole 111 has an opening towards the interior of the balloon; the liner core is slidably inserted into the pushing tube, the distal end part of the liner core 4 is a curved section 41, which protrudes out of the distal end of the pushing tube 2, the distal end of the liner core 4 is inserted into the blind hole 111 of the sealing head 11 through the pushing tube 2 and the interior of said balloon 1, and the balloon 1 wraps up the curved section 41 of the liner core 4; the distal end part of the sleeve tube 3 is a flexible section 31, whereas the proximal end part thereof is a rigid section 32, the flexible section 31 is rigid in the radial direction, and the sleeve tube 3 is sleeved on the pushing tube 2 and the balloon 1. In the operation of the vertebral balloon dilation system, the sleeve tube 3 slides towards the proximal end of the pushing tube 2 in the axial direction, and the balloon 1 is exposed.

As shown in Fig. 2, the distal end of the balloon 1 is fixedly connected with a sealing head 11, the distal end of which is closed, and the sealing head 11 is made of a rigid material. In one embodiment, as shown in Fig. 2a, the proximal end of the sealing head 11 is provided with a blind hole 111 with an opening towards the interior of said balloon. The head end of the sealing head 11 is in a sharp shape, and the sealing head 11 is provided with a ring-shaped groove 112. The distal end of the balloon 1 is tightly bound to the ring-shaped groove 112 of the sealing head 11 by means of wires (threads) 113, and the distal end of the balloon 1 is turned over and wraps up the wires 113. In another embodiment, as shown in Fig. 2b, the sealing head 11 is provided with an inner core 112', the proximal end of which is provided with an external thread, and the inner core 112' has a blind hole 111. The blind hole 111 has an opening towards the interior of the balloon 1, and the distal end of the sealing head 11 is in a sharp shape, and the sealing head 11 is provided with an internal thread. The external thread of the inner core 112 and the internal thread of the sealing head 11 are both trapezoidal threads, and the distal end of the balloon 1 is tightly clamped between the external thread of the inner core 112' and the internal thread of the sealing head 11 such that the threaded connection is formed. In a third embodiment, as shown in Figs. 2c and 2d, the sealing head 11 is provided with a fixing ring 112", wherein the distal end of the balloon 1 passes through the inner hole of the fixing ring 112" and then is turned over outwardly to wrap up the fixing ring 112", and the proximal end of the sealing head 11 has several pieces 114 formed by cutting. After the distal end of the balloon 1 is turned over to wrap up the fixing ring 112", it is covered with the sealing head 11, and then the pieces 114 provided at the proximal end of the sealing head 11 are pressed down to fixedly connect the distal end of the balloon 1 with the sealing head 11 and meanwhile the interior of the fixing ring 112" forms the blind hole 111.

In one embodiment, as shown in Figs. 3a and 3b, the balloon 1 is formed in a banana shape or a dumbbell shape, and the balloon 1 is made of PTFE (polytetrafluoroethylene) membrane.

As shown in Figs. 3 and 4, the proximal end of the balloon 1 is fixedly connected to the outside of the distal end of the pushing tube 2 by means of hot melt welding or sintering. In one embodiment, as shown in Figs. 3a and 3b, the proximal end of the balloon 1 is hot-melt welded to the distal end of the pushing tube 2. In another embodiment, as shown in Figs. 4a and 4b, the proximal end part of the balloon 1 extends to form a tubular section 12, which is entirely sintered to the external surface of the distal end of the pushing tube 2.

As shown in Figs. 1c and 1e, in one embodiment, the distal end part of the liner core 4 is a curved section 41 with the shape memory property. The curved section 41 is made of nitinol wires (as shown in Fig. 1c) or a combination of nitinol wires 411 and polymer material 412 (as shown in Fig. 1e). The end part of the curved section 41 is a straight section 42, which can be slidably inserted to the blind hole 111 of the sealing head 11 and finally reach the bottom of the blind hole 111. After the straight section 42 is inserted into the blind hole 111, the sealing head 11 will not be driven when the liner core 4 is withdrawn, such that the displacement of the balloon 1 can be prevented.

As shown in Fig. 5, the balloon 1 is foldable in the axial direction thereof, and wraps up the curved section of the liner core 4 along the circumferential direction thereof.

As shown in Figs. 1c, 6a and 6b, the vertebral balloon dilation system further comprises a liner core handle 5 fixedly connected to the proximal end of the liner core 4, a pushing handle 6 fixedly connected to the proximal end of the pushing tube 2, and a sleeve tube handle 7 connected to the proximal end of the sleeve tube 3, wherein the proximal end of the pushing handle 6 is provided with a port in communication with a balloon filling device, the pushing handle 6 is provided with one or more guide grooves 61, the distal end of the pushing handle 6 is provided with a stop block 62 for limiting the position of the sleeve tube handle 7, and the sleeve tube handle 7 is provided with one or more guide blocks 71 engaged with the guide grooves 61 of the pushing handle 6, such that, when the sleeve tube handle 7 is moved, the sleeve tube 3 can move in the axial direction together with the sleeve tube handle 7.

As shown in Figs. 1 and 5, the distal end part of the sleeve tube 3 is a flexible section 31, whereas the proximal end part thereof is a rigid section 32, wherein the flexible section 31 is rigid in the radial direction, the sleeve tube 3 is sleeved on the pushing tube 2 and the balloon 1 (as shown in Fig. 1b), and the distal end of the sleeve tube 3 is sleeved on or connected to the sealing head 11 (as shown in Fig. 5). When the sleeve tube handle 7 is operated, the sleeve tube 3 is moved axially to the proximal end of the pushing tube 2, and the balloon 1 is exposed. As shown in Figs. 7-11, the flexible section of the sleeve tube 3 is cut from a metal tube, formed of a spring, or made of a hard medical polymer material or any combinations of metal materials or polymer materials. In one embodiment, as shown in Figs. 7a and 7b, the flexible section 31 of the sleeve tube 3 is cut from a metal tube, and the rigid section 32 is a metal tube. In another embodiment, as shown in Fig. 8, the flexible section 31 of the sleeve tube 3 is formed of a metal spring, and the rigid section 32 is a metal tube, wherein the proximal end of the flexible section 31 is welded to the distal end of the rigid section 32. In a third embodiment, as shown in Figs. 9a and 9b, the flexible section 31 of the sleeve tube 3 is formed with a curved section made of a medical polymer material (preferably, PEEK) by heat-setting. In a fourth embodiment, as shown in Figs. 10a and 10b, the flexible section 31 of the sleeve tube 3 is made of a combination of metal material 311 and medical polymer material 312, and the rigid section 32 is made of a metal material.

According to above-mentioned embodiments, the sealing head 11, the inner core 112' and the fixing ring 112" are made of a metal material (preferably, pure titanium) or a hard medical polymer material (preferably, PEEK). The balloon 1 is made of a medical polymer material (preferably, expanded PTFE). The pushing tube 2 is formed of a metal tube (preferably, 304 stainless steel), the flexible section 31 of the sleeve tube 3 is cut from a metal tube, formed of a spring or shaped from a medical polymer material (preferably, PEEK), and the rigid section 32 of the sleeve tube 3 is formed of a metal tube or made of a medical polymer material (preferably, PEEK). The liner core 4 is made of shape memory alloy (preferably, nitinol). The sleeve tube handle 7, the pushing handle 6 and the liner core handle 5 are made of a medical polymer material (preferably, POM).

In surgical procedures, after a working channel is established, the vertebral balloon dilation system is inserted into the vertebral body through the working channel up to a predetermined position under the observation with a C-arm, and during the procedure, the liner core 4 has the following functions:
1. The distal end of the liner core 4 abuts against the blind hole 111 of the sealing head 11, such that the balloon 1 can be prevented from moving or turning over in the sleeve tube 3 during the feeding procedure, and the sealing head 11 can be driven to push away the bone tissue by pushing the liner core 4.
2. The balloon 1 is configured to wrap up the curved section 41 of the liner core 4 such that the balloon can be fed to the center or the opposite side of the vertebral body under the protection of the sleeve tube 3.

As shown in Figs. 11a and 11b, after the vertebral balloon dilation system is inserted to the predetermined position, the sleeve tube handle 7 is moved towards the proximal end to make the sleeve tube 3 move backwards slowly until the balloon 1 is exposed entirely, at this time, the liner core handle 5 is pulled to withdraw the liner core 4 completely, and after that, the balloon filling device is coupled to the pushing handle 6 to inject developer solution to the balloon 1 through the pushing tube 2 until the balloon 1 is dilated. After the balloon 1 has been dilated completely, a negative pressure is formed in the balloon 1, then the balloon is withdrawn out of the body, and finally, the bone cement is injected to the vertebral body through the working channel.

## Claims

1. A vertebral balloon dilation system, comprising a pushing tube (2) and a balloon (1) fixedly connected to a distal end of said pushing tube (2), wherein:
said vertebral balloon dilation system can withstand water pressure of no less than 10 atm; said pushing tube (2) is a rigid tube, a lumen of said pushing tube (2) is in communication with an interior of said balloon (1); and
said balloon (1) is made of a membrane material, a distal end of said balloon is fixedly connected with a sealing head (11) that is closed at its distal end and has a blind hole (111) at its proximal end, wherein said blind hole has an opening towards the interior of said balloon;
**characterized in that**:
said vertebral balloon dilation system further comprises a sleeve tube (3) and a liner core (4), wherein said liner core (4) is slidably inserted into said pushing tube (2), a distal end part of said liner core (4) is a curved section (41), which protrudes out of the distal end of said pushing tube (2), a distal end of said liner core (4) is inserted into said blind hole (111) of said sealing head (11) through said pushing tube (2) and the interior of said balloon (1), and said balloon (1) is configured to wrap up the curved section (41) of the liner core (4); a distal end part of said sleeve tube (3) is a flexible section (31), whereas a proximal end part of said sleeve tube is a rigid section (32), wherein said flexible section (31) is rigid in the radial direction, and said sleeve tube (3) is sleeved on said pushing tube (2) and said balloon (1); and
when said vertebral balloon dilation system is operated, said sleeve tube (3) slides towards a proximal end of said pushing tube (2) in the axial direction, and said balloon (1) is exposed.

2. The vertebral balloon dilation system according to claim 1, **characterized in that**: said sealing head (11) is connected with said balloon (1) in a connection manner selected from binding connection, threaded connection, compression connection, adhesive connection, or welding connection or combinations thereof.

3. The vertebral balloon dilation system according to claim 1, **characterized in that**: said membrane material is polytetrafluoroethylene membrane.

4. The vertebral balloon dilation system according to claim 1, **characterized in that**: said sealing head (11) is made of a rigid material, the distal end of said sealing head is in a sharp shape, and the distal end of said sleeve tube (3) is sleeved on or connected with said sealing head (11).

5. The vertebral balloon dilation system according to claim 1, **characterized in that**: an end part of said curved section (41) of said liner core (4) is a straight section (42), which is inserted into said blind hole (111) in said sealing head (11) and reaches the bottom of said blind hole (111).

6. The vertebral balloon dilation system according to claim 1, **characterized in that**: said flexible section (31) of said sleeve tube (3) is cut from a metal tube, formed of a spring, made of a hard medical polymer material, or formed of any combinations of metal materials and polymer materials.

7. The vertebral balloon dilation system according to claim 1, **characterized in that**: said balloon is in a banana shape or a dumbbell shape.

8. The vertebral balloon dilation system according to claim 1, **characterized in that**: said balloon (1) is foldable in its axial direction, and is configured to wrap up said curved section (41) of the liner core (4) along its circumferential direction.

9. The vertebral balloon dilation system according to claim 1, **characterized in that**: said vertebral balloon dilation system further comprises a liner core handle (5) fixedly connected to the proximal end of said liner core (4), a pushing handle (6) fixedly connected to the proximal end of said pushing tube (2), and a sleeve tube handle (7) connected to the proximal end of said sleeve tube (3), wherein said pushing handle (6) includes one or more guide grooves (61), a distal end of said pushing handle (6) is provided with a stop block (62) for limiting the position of said sleeve tube handle (7), and said sleeve tube handle (7) is provided with one or more guide blocks (71) engaged with said guide grooves (61) such that while said sleeve tube handle (7) is moved, said sleeve tube (3) will move in the axial direction together with said sleeve tube handle (7).

10. The vertebral balloon dilation system according to claim 1, **characterized in that**: the distal end of said pushing tube (2) and said balloon (1) are fixedly connected in a manner of hot melt welding, or sintering the proximal end of said balloon (1) entirely to an outside of said pushing tube (2).

## Patentansprüche

1. Vertebrales Ballondilatationssystem, umfassend ein Schieberohr (2) und einen Ballon (1), der fest mit einem distalen Ende des Schieberohrs (2) verbunden ist, wobei:
das vertebrale Ballondilatationssystem einem Wasserdruck von nicht weniger als 10 atm standhalten kann; das Schieberohr (2) ein starres Rohr ist, ein Lumen des Schieberohrs (2) mit einem Inneren des Ballons (1) in Verbindung steht; und
der Ballon (1) aus einem Membranmaterial hergestellt ist, wobei ein distales Ende des Ballons fest mit einem Dichtungskopf (11) verbunden ist, der an seinem distalen Ende geschlossen ist und an seinem proximalen Ende ein Sackloch (111) aufweist, wobei das Sackloch eine Öffnung zum Inneren des Ballons hin aufweist;
**dadurch gekennzeichnet, dass**:
das vertebrale Ballondilatationssystem ferner ein Hülsenrohr (3) und einen Auskleidungseinsatz (4) umfasst, wobei der Auskleidungseinsatz (4) verschiebbar in das Schieberohr (2) eingeführt ist, ein distales Endteil des Auskleidungseinsatzes (4) ein gekrümmter Abschnitt (41) ist, der aus dem distalen Ende des Schieberohrs (2) herausragt, ein distales Ende des Auskleidungseinsatzes (4) in das Sackloch (111) des Dichtungskopfes (11) durch das Schieberohr (2) und das Innere des Ballons (1) eingeführt wird und der Ballon (1) so konfiguriert ist, dass er den gekrümmten Abschnitt (41) des Auskleidungseinsatzes (4) umhüllt; ein distaler Endabschnitt des Hülsenrohrs (3) ein flexibler Abschnitt (31) ist, während hingegen ein proximaler Endabschnitt des Hülsenrohrs ein starrer Abschnitt (32) ist, wobei der flexible Abschnitt (31) in radialer Richtung starr ist und das Hülsenrohr (3) auf das Schieberohr (2) und den Ballon (1) aufgesteckt ist; und
wenn das vertebrale Ballondilatationssystem betrieben wird, das Hülsenrohr (3) zu einem proximalen Ende des Schieberohres (2) in axialer Richtung gleitet und der Ballon (1) freigelegt wird.

2. Vertebrales Ballondilatationssystem nach Anspruch 1, **dadurch gekennzeichnet, dass**: der Dichtungskopf (11) mit dem Ballon (1) in einer Verbindungsart verbunden ist, die aus Formschlussverbindung, Gewindeverbindung, Pressverbindung, Klebeverbindung oder Schweißverbindung oder Kombinationen davon ausgewählt ist.

3. Vertebrales Ballondilatationssystem nach Anspruch 1, **dadurch gekennzeichnet, dass**: das Membranmaterial eine Polytetrafluorethylenmembran ist.

4. Vertebrales Ballondilatationssystem nach Anspruch 1, **dadurch gekennzeichnet, dass**: der Dichtungskopf (11) aus einem starren Material hergestellt ist, das distale Ende des Dichtungskopfes eine spitze Form aufweist und das distale Ende des Hülsenrohres (3) auf den Dichtungskopf (11) aufgesteckt oder mit diesem verbunden ist.

5. Vertebrales Ballondilatationssystem nach Anspruch 1, **dadurch gekennzeichnet, dass**: ein Endabschnitt des gekrümmten Abschnitts (41) des Auskleidungseinsatzes (4) ein gerader Abschnitt (42) ist, der in das Sackloch (111) in dem Dichtungskopf (11) eingeführt ist und den Boden des Sacklochs (111) erreicht.

6. Vertebrales Ballondilatationssystem nach Anspruch 1, **dadurch gekennzeichnet, dass**: der flexible Abschnitt (31) des Hülsenrohres (3) aus einem Metallrohr geschnitten ist, aus einer Feder gebildet ist, aus einem harten medizinischen Polymermaterial hergestellt ist oder aus beliebigen Kombinationen von Metallmaterialien und Polymermaterialien gebildet ist.

7. Vertebrales Ballondilatationssystem nach Anspruch 1, **dadurch gekennzeichnet, dass**: der Ballon eine Bananenform oder eine Hantelform aufweist.

8. Vertebrales Ballondilatationssystem nach Anspruch 1, **dadurch gekennzeichnet, dass**: der Ballon (1) in seiner axialen Richtung faltbar ist und so konfiguriert ist, dass er den gekrümmten Abschnitt (41) des Auskleidungseinsatzes (4) entlang seiner Umfangsrichtung umhüllt.

9. Vertebrales Ballondilatationssystem nach Anspruch 1, **dadurch gekennzeichnet, dass**: das vertebrale Ballondilatationssystem ferner einen Auskleidungseinsatz-Griff (5), der fest mit dem proximalen Ende des Auskleidungseinsatzes (4) verbunden ist, einen Schiebegriff (6), der fest mit dem proximalen Ende des Schieberohrs (2) verbunden ist, und einen Hülsenrohrgriff (7) umfasst, der mit dem proximalen Ende des Hülsenrohrs (3) verbunden ist, wobei der Schiebegriff (6) eine oder mehrere Führungsnuten (61) umfasst, wobei ein distales Ende des Schiebegriffs (6) mit einem Anschlagblock (62) zum Begrenzen der Position des Hülsenrohrgriffs (7) versehen ist, und der Hülsenrohrgriff (7) mit einem oder mehreren Führungsblöcken (71) versehen ist, die mit den Führungsnuten (61) derart in Eingriff stehen, dass sich das Hülsenrohr (3), während der Hülsenrohrgriff (7) bewegt wird, zusammen mit dem Hülsenrohrgriff (7) in axialer Richtung bewegt.

10. Vertebrales Ballondilatationssystem nach Anspruch 1, **dadurch gekennzeichnet, dass**: das distale Ende des Schieberohres (2) und des Ballons (1) fest mittels Klebeverschweißung oder Sintern des proximalen Endes des Ballons (1) vollständig mit einer Außenseite des Schieberohres (2) verbunden sind.

## Revendications

1. Système de dilatation vertébrale à ballonnet comprenant un tube de poussée (2) et un ballonnet (1) raccordé, de manière fixe, à une extrémité distale dudit tube de poussée (2), dans lequel :
ledit système de dilatation vertébrale à ballonnet peut résister à la pression de l'eau d'une valeur non inférieure à 10 atm ; ledit tube de poussée (2) est un tube rigide, une lumière dudit tube de poussée (2) est en communication avec un intérieur dudit ballonnet (1) ; et
ledit ballonnet (1) est réalisé avec un matériau de membrane, une extrémité distale dudit ballonnet est raccordée, de manière fixe, avec une tête d'étanchéité (11) qui est fermée au niveau de son extrémité distale et a un trou borgne (111) au niveau de son extrémité proximale, dans lequel ledit trou borgne a une ouverture vers l'intérieur dudit ballonnet ;
**caractérisé en ce que** :
ledit système de dilatation vertébrale à ballonnet comprend en outre un tube de manchon (3) et un noyau de revêtement (4), dans lequel ledit noyau de revêtement (4) est inséré, de manière coulissante, dans ledit tube de poussée (2), une partie d'extrémité distale dudit noyau de revêtement (4) est une section incurvée (41) qui fait saillie de l'extrémité distale dudit tube de poussée (2), une extrémité distale dudit noyau de revêtement (4) est insérée dans ledit trou borgne (111) de ladite tête d'étanchéité (11) à travers ledit tube de poussée (2) et l'intérieur dudit ballonnet (1), et ledit ballonnet (1) est configuré pour envelopper la section incurvée (41) du noyau de revêtement (4) ; une partie d'extrémité distale dudit tube de manchon (3) est une section flexible (31), alors qu'une partie d'extrémité proximale dudit tube de manchon est une section rigide (32), dans lequel ladite section flexible (31) est rigide dans la direction radiale, et ledit tube de manchon (3) est emmanché sur ledit tube de poussée (2) et ledit ballonnet (1) ; et
lorsque ledit système de dilatation vertébrale à ballonnet est actionné, ledit tube de manchon (3) coulisse vers une extrémité proximale dudit tube de poussée (2) dans la direction axiale, et ledit ballonnet (1) est exposé.

2. Système de dilatation vertébrale à ballonnet selon la revendication 1, **caractérisé en ce que** : ladite tête d'étanchéité (11) est raccordée avec ledit ballonnet (1) par raccordement sélectionné parmi un raccordement de liaison, un raccordement fileté, un raccordement par compression, un raccordement adhésif ou un raccordement par soudage ou leurs combinaisons.

3. Système de dilatation vertébrale à ballonnet selon la revendication 1, **caractérisé en ce que** : ledit matériau de membrane est une membrane en polytétrafluoroéthylène.

4. Système de dilatation vertébrale à ballonnet selon la revendication 1, **caractérisé en ce que** : ladite tête d'étanchéité (11) est réalisée avec un matériau rigide, l'extrémité distale de ladite tête d'étanchéité se présente sous une forme tranchante, et l'extrémité distale dudit tube de manchon (3) est emmanchée sur ou raccordée avec ladite tête d'étanchéité (11).

5. Système de dilatation vertébrale à ballonnet selon la revendication 1, **caractérisé en ce que** : une partie d'extrémité de ladite section incurvée (41) dudit noyau de revêtement (4) est une section droite (42), qui est insérée dans ledit trou borgne (111) dans ladite tête d'étanchéité (11) et atteint le fond dudit trou borgne (111) .

6. Système de dilatation vertébrale à ballonnet selon la revendication 1, **caractérisé en ce que** : ladite section flexible (31) dudit tube de manchon (3) est coupée dans un tube métallique, formé avec un ressort, réalisé dans un matériau polymère dur de qualité médicale, ou formé avec n'importe quelle combinaison de matériaux métalliques et de matériaux polymères.

7. Système de dilatation vertébrale à ballonnet selon la revendication 1, **caractérisé en ce que** : ledit ballonnet se présente sous la forme d'une banane ou sous la forme d'une altère.

8. Système de dilatation vertébrale à ballonnet selon la revendication 1, **caractérisé en ce que** : ledit ballonnet (1) peut être plié, dans sa direction axiale, et est configuré pour envelopper ladite section incurvée (41) du noyau de revêtement (4) le long de sa direction circonférentielle.

9. Système de dilatation vertébrale à ballonnet selon la revendication 1, **caractérisé en ce que** : ledit système de dilatation vertébrale à ballonnet comprend en outre une poignée de noyau de revêtement (5) raccordée, de manière fixe, à l'extrémité proximale dudit noyau de revêtement (4), une poignée de poussée (6) raccordée, de manière fixe, à l'extrémité proximale dudit tube de poussée (2), et une poignée de tube de manchon (7) raccordée à l'extrémité proximale dudit tube de manchon (3), dans lequel ladite poignée de poussée (6) comprend une ou plusieurs rainures de guidage (61), une extrémité distale de ladite poignée de poussée (6) est prévue avec un bloc de butée (62) pour limiter la position de ladite poignée de tube de manchon (7), et ladite poignée de tube de manchon (7) est prévue avec un ou plusieurs blocs de guidage (71) mis en prise avec lesdites rainures de guidage (61), de sorte que, alors que ladite poignée de tube de manchon (7) est déplacée, ledit tube de manchon (3) se déplace dans la direction axiale conjointement avec ladite poignée de tube de manchon (7).

10. Système de dilatation vertébrale à ballonnet selon la revendication 1, **caractérisé en ce que** : l'extrémité distale dudit tube de poussée (2) et ledit ballonnet (1) sont raccordés, de manière fixe, par soudage thermofusible ou en frittant l'extrémité proximale dudit ballonnet (1) entièrement sur l'extérieur dudit tube de poussée (2).
